# DEMANDE DE BREVET EUROPEEN

(11) **EP 1 875 894 A2**
(43) Date de publication de la demande: **09.01.2008**
(21) Numéro de dépôt: 07301199.1
(22) Date de dépôt: 02.07.2007
(51) Int. Cl.: A61K 8/60, A61Q 19/00, A61K 31/70

(54) **Utilisation d'au moins un dérivé C-glycoside à titre d'agent apaisant**

(30) Priorité: 03.07.2006 FR 0606015
(71) Demandeur: L'ORÉAL, 75008 Paris (FR)
(72) Inventeur: WILLEMIN, Claudie, 75008, PARIS (FR); BURNIER, Véronique, 91210, DRAVEIL (FR)
(74) Mandataire: Le Coupanec, Pascale A.M.P.

(57) **Abrégé**

La présente invention concerne une utilisation non thérapeutique d'un dérivé C-glycoside à titre d'agent apaisant.

## Description

La présente invention a pour objet l'utilisation non thérapeutique d'au moins un dérivé C-glycoside à titre d'agent apaisant, ainsi que l'utilisation d'au moins un dérivé C-glycoside pour la préparation d'une composition destinée à prévenir et/ou traiter les désordres cutanés liés à une irritation cutanée.

La présente invention concerne en outre une composition cosmétique ou dermatologique comprenant l'association d'au moins un composé cosmétique ou pharmaceutique susceptible de provoquer une irritation de la peau et d'au moins un dérivé C-glycoside.

Enfin, la présente invention concerne un procédé cosmétique comprenant au moins une étape consistant à appliquer sur la peau, une muqueuse, le cuir chevelu et/ou les cheveux une composition selon l'invention.

Des réactions cutanées peuvent se manifester sous forme d'inconfort ressenti au niveau de la peau par l'utilisateur. Il peut typiquement s'agir de rougeurs, de démangeaisons, d'échauffement, de sensations de brûlures, de sensations de picotement, de tiraillement. Des traitements cosmétiques permettent de remédier à cet inconfort.

D'autres signes cutanés visibles peuvent également apparaître nécessitant avantageusement cette fois un traitement dermatologique. Parmi ces signes cutanés visibles on peut notamment citer les prurits, les dartres, les érythèmes inflammatoires, les oedèmes et/ou boutons, ou encore les réactions cutanées d'irritation.

Ces réactions cutanées, notamment l'irritation cutanée, peuvent aussi être induites par un stress exogène, d'origine chimique, par exemple, xénobiotiques, antigènes, allergènes, produits chimiques, composés susceptibles de provoquer une irritation de la peau, peeling, d'origine environnementale (température, climat, rayonnement UV, pollution atmosphérique, notamment métaux lourds, ozone, fumée de cigarette...) ou encore d'origine mécanique (frottements, rasage) et tout stress d'origine endogène tels que les désordres impliquant un mécanisme inflammatoire et/ou hormonal affectant la peau, une muqueuse, le cuir chevelu et/ou les cheveux.

Le stress endogène physiologique peut par exemple être lié à la production anormale de médiateurs proinflammatoires (neuromediateurs, cytokine, chemokines) ou à une alopécie androgénétique.

L'irritation cutanée met en oeuvre une cascade de réactions qui, par le recrutement de cellules sanguines infiltrantes (neutrophiles, macrophages, cellules de Langherans) et les substances qu'elles libèrent (cytokines, lymphokines, chemokines...), donnent naissance au processus irritant persistant qui se caractérise principalement par une irritation de la peau ou une involution du bulbe pilaire et de son environnement matriciel.

Des composés topiques dont l'utilisation peut, dans des circonstances particulières telles qu'une peau réactive, une peau atteinte de rosacée, des concentrations élevées desdits composés..., conduire à l'apparition de réactions cutanées, sont utilisés dans des compositions cosmétiques ou dermatologiques, bien entendu pour d'autres effets.

Ainsi, on utilise des compositions cosmétiques contenant par exemple des actifs kératolytiques et/ou desquamants, pour lutter contre le vieillissement, et notamment des actifs exfoliants et /ou des actifs favorisant le renouvellement cellulaire, tels que les α-hydroxy-acides (notamment acides lactique, glycolique, citrique), les β-hydroxy-acides (notamment acides salicylique, n-octanoyl-5-salicylique) et les rétinoïdes (notamment acide rétinoïque tout trans ou 13-cis, rétinol). Malheureusement, si ces actifs sont utilisés en des quantités trop importantes, ils peuvent provoquer une irritation cutanée. L'utilisation de ces composés notamment pour les utilisateurs à peaux et/ou cuirs chevelus irritables et/ou allergiques doit donc être limitée.

En outre, même certains composés qui sont considérés comme inertes dans une composition cosmétique ou dermatologique, tels que, par exemple, les conservateurs, les tensioactifs, les parfums, les solvants ou les propulseurs, peuvent présenter un caractère irritant lorsqu'ils sont appliqués sur les matières kératiniques et notamment la peau y compris le cuir chevelu chez des sujets à peau irritable, sensible et/ou allergique, ce caractère irritant dépendant du composé utilisé et de la sensibilité de la peau et de la flore cutanée résidente de l'utilisateur.

Les composés susceptibles de provoquer une irritation de la peau sont généralement utilisés en des doses faibles. L'utilisation à faible quantité de ces composés peut alors s'avérer peu avantageuse par rapport à l'utilisation d'autres composés moins actifs, mais moins ou pas irritants et donc utilisés en plus grande quantité, ou par rapport à la finalité du composé, telle que par exemple, la stabilité de la composition quand il s'agit d'émulsionnants ou la bonne conservation de la composition quand il s'agit de conservateurs.

On sait également que certains désordres cutanés sont liés à une réaction cutanée de type inflammatoire ou immuno-allergique, parmi lesquels l'érythème inflammatoire, le psoriasis, l'atopie cutanée, la dermatite atopique, les réactions allergiques de type hypersensibilité immédiate, telles que l'urticaire, les réactions allergiques de type hypersensibilité retardée, telles que les dermites de contact, l'eczéma ; les dermatites séborrhéiques, l'acné, les hyperpigmentations inflammatoires, les dermatoses immunes, l'élastose actinique, les pelades ou *alopecia areata* ; le vitiligo ; le lupus érythémateux systémique ; *le pemphigus vulgaris;* les épidermolyses bulbeuses dystrophiques et la canitie d'origine autoimmune.

Il existe donc un besoin de trouver des composés à effet apaisant capables notamment de prévenir et/ou diminuer l'effet irritant de compositions cosmétiques ou dermatologiques contenant un ou plusieurs composés susceptibles de provoquer une irritation de la peau, et de prévenir et/ou traiter des désordres cutanés liés à une irritation cutanée de type inflammatoire ou immuno-allergique.

Les inventeurs ont découvert de façon surprenante que certains dérivés C-glycosides démontrent des propriétés apaisantes qui justifient notamment de leur intérêt à les incorporer dans des compositions comprenant au moins un composé susceptible de provoquer une irritation de la peau.

Les sucres et dérivés du sucre sont des produits déjà mis à profit à des fins diverses pour la formulation de compositions cosmétiques destinées tant au soin de la peau qu'au soin et/ou lavage des fibres kératiniques.

En particulier, certains dérivés C-glycosides sont déjà connus pour manifester des propriétés biologiques intéressantes, en particulier pour lutter contre le vieillissement de l'épiderme et/ou contre le dessèchement de la peau. De tels composés sont notamment décrits dans le document WO 02/051828.

Ainsi, l'invention concerne l'utilisation non thérapeutique, en particulier l'utilisation cosmétique, d'au moins un dérivé C-glycoside à titre d'agent apaisant notamment d'agent apaisant topique.

Elle a ainsi pour objet ladite utilisation non thérapeutique d'au moins un dérivé C-glycoside selon la présente invention, destinée à prévenir et/ou traiter les réactions cutanées choisies parmi les rougeurs, les démangeaisons, les échauffements, les sensations de brûlure, de picotement et de tiraillement.

L'invention a de plus pour objet ladite utilisation selon la présente invention, caractérisée en ce que ledit dérivé C-glycoside est destiné à prévenir et/ou diminuer ladite réaction cutanée induite par au moins une condition choisie parmi l'action de xénobiotiques, de produits chimiques, de composés susceptibles de provoquer une irritation de la peau, d'un peeling, l'action de la température, du climat, de rayonnements UV, de la pollution atmosphérique, ou encore par les frottements.

Le dérivé C-glycoside est alors avantageusement destiné à prévenir et/ou diminuer l'effet irritant d'une composition cosmétique ou dermatologique contenant un ou plusieurs composés susceptibles de provoquer une irritation.

L'invention se rapporte en outre à l'utilisation non thérapeutique, en particulier cosmétique, d'un dérivé C-glycoside pour le traitement des peaux et/ou cuirs chevelus sensibles.

Elle se rapporte de plus à l'utilisation non thérapeutique, en particulier cosmétique d'un dérivé C-glycoside pour le traitement des peaux et/ou cuirs chevelus sensibles, caractérisée en ce qu'elle est destinée au traitement des rougeurs et/ou des dartres et/ou des sensations dysesthésiques choisies parmi picotements, fourmillements, démangeaisons, brûlures, échauffements, inconforts, tiraillements des peaux et/ou cuirs chevelus sensibles.

Elle concerne également l'utilisation non thérapeutique d'un dérivé C-glycoside pour le traitement des peaux et/ou cuirs chevelus sensibles, caractérisée en ce que le dérivé C-glycoside est associé à un extrait de rose, notamment un extrait de rose tel que détaillé ci-après.

L'invention a encore pour objet l'utilisation d'au moins un dérivé C-glycoside pour la préparation d'une composition dermatologique destinée à prévenir et/ou traiter les désordres cutanés liés à une irritation cutanée, par exemple rencontrée chez les sujets présentant des peaux et/ou muqueuses et/ou cuirs chevelus irritables et/ou allergiques.

L'invention se rapporte donc aux dérivés C-glycosides pour leur utilisation pour le traitement des désordres cutanés liés à une irritation cutanée, par exemple rencontrée chez les sujets présentant des peaux et/ou muqueuses et/ou cuirs chevelus irritables et/ou allergiques.

L'invention a plus particulièrement pour objet ladite utilisation pour la préparation d'une composition dermatologique destinée à prévenir et/ou traiter l'irritation cutanée, caractérisée en ce que l'irritation cutanée est induite par au moins une condition choisie parmi l'action de xénobiotiques, de produits chimiques, de composés susceptibles de provoquer une irritation de la peau, d'un peeling, l'action de la température, du climat, de rayonnements UV, de la pollution atmosphérique, ou encore par les frottements.

L'invention concerne plus particulièrement également ladite utilisation d'au moins un dérivé C-glycoside pour la préparation d'une composition dermatologique, caractérisée en ce que ledit dérivé C-glycoside est destiné à prévenir et/ou diminuer l'effet irritant d'une composition cosmétique ou dermatologique contenant un ou plusieurs composés susceptibles de provoquer une irritation de la peau.

L'invention a également pour objet l'utilisation d'au moins un dérivé C-glycoside pour la préparation d'une composition dermatologique destinée à prévenir et/ou traiter des désordres cutanés liés à une réaction cutanée, par exemple de type inflammatoire ou immuno-allergique.

L'invention se rapporte donc aussi aux dérivés C-glycosides pour leur utilisation pour le traitement des désordres cutanés liés à une réaction cutanée, par exemple de type inflammatoire ou immuno-allergique.

Ainsi, les dérivés C-glycosides sont plus particulièrement utiles pour lutter contre les désordres cutanés choisis parmi les dartres, les oedèmes et/ou boutons, l'érythème inflammatoire ; les prurits ; le psoriasis, l'atopie cutanée, la dermatite atopique ; l'urticaire; les dermites de contact, l'eczéma ; les dermatites séborrhéiques ; l'acné ; les hyperpigmentations inflammatoires ; les dermatoses immunes ; les maladies immunes bulleuses ; la sclérodermie ; l'élastose actinique ; les pelades ou *alopecia areata ;* le vitiligo ; le lupus érythémateux systémique ; le *pemphigus vulgaris ;* les épidermolyses bulbeuses dystrophiques et la canitie d'origine autoimmune.

Que ce soit à des fins cosmétiques ou thérapeutiques, l'utilisation selon la présente invention est particulièrement destinée au traitement des peaux et/ou muqueuses et/ou cuirs chevelus sensibles et/ou irritables et/ou allergiques.

L'invention a en outre pour objet une composition pour application topique sur la peau et/ou les muqueuses et/ou le cuir chevelu comprenant, dans un milieu physiologiquement acceptable :
- au moins un composé cosmétique ou pharmaceutique susceptible de provoquer une irritation de la peau choisi parmi les agents desquamants, les rétinoïdes, l'urée et ces dérivés, la vitamine D et ses dérivés, la vitamine B9 et ses dérivés, les peroxydes, les antichutes, les teintures et les colorants capillaires, les agents anti-transpirants, les déodorants, les actifs dépilatoires et/ou de permanentes, le thioglycolate et ses sels, le phénoxyéthanol, le 1,2-pentanediol, les anthralines, les anthranoïdes, les sels de lithium, les dépigmentants, certains actifs amincissants à effet chauffant, les nicotinates et leurs dérivés, la capsaïcine, les actifs antipoux, les antiprolifératifs, les agents antiviraux, les antiparasitaires, les antifongiques, les antiprurigineux, les antiséborrhéiques, certaines filtres solaires, les propigmentants, les alkyl sulfates et alkyl éther sulfates, les tensioactifs, cationiques ou amphotères et,
- au moins un dérivé C-glycoside.

Dans une variante de l'invention, le composé cosmétique ou pharmaceutique susceptible de provoquer une irritation de la peau n'est ni un agent kératolytique, ni un agent desquamant.

L'invention a encore pour objet une composition pour application topique sur la peau et/ou les muqueuses et/ou le cuir chevelu comprenant dans un milieu physiologiquement acceptable :
- 0,00001 % à 95 % en poids d'au moins un composé anti-inflammatoire et/ou apaisant et,
- au moins un dérivé C-glycoside.

L'invention a aussi pour objet un procédé cosmétique pour le soin non thérapeutique de la peau, des cheveux et/ou du cuir chevelu, caractérisé en ce qu'il comprend au moins une étape consistant à appliquer sur la peau, les cheveux et/ou le cuir chevelu une composition cosmétique selon la présente invention.

L'invention a enfin pour objet un procédé cosmétique pour le soin non thérapeutique de la peau, des cheveux et/ou du cuir chevelu mettant en oeuvre au moins une composition cosmétique selon la présente invention, particulièrement adaptée au traitement des peaux et/ou muqueuses et/ou cuirs chevelus sensibles et/ou irritables et/ou allergiques.

Ce procédé cosmétique selon l'invention est notamment destiné à prévenir et/ou traiter les signes du vieillissement cutané, et/ou à éclaircir la peau, et/ou à raviver l'éclat du teint, et/ou à stimuler la croissance des cheveux ou freiner leur chute.

Selon une variante de l'invention, le procédé cosmétique est destiné à apaiser la peau, en particulier, à prévenir et/ou traiter les manifestations cutanées choisies parmi les rougeurs, les démangeaisons, les échauffements, les sensations de brûlure, de picotement et de tiraillement.

Plus particulièrement, le procédé selon l'invention est destiné à apaiser les peaux des individus à qui un produit susceptible de provoquer des irritations cutanées a été administré, notamment appliqué topiquement.

Par traiter, sauf indication contraire, on entend toute action visant à améliorer le confort, le bien-être d'un individu, ce terme couvre donc aussi bien prévenir, atténuer, soulager que curer.

L'utilisation d'au moins un dérivé C-glycoside présente donc entre autre l'avantage de supprimer l'irritation cutanée qu'auraient pu provoquer les composés à effet secondaire irritant, et aussi de permettre d'augmenter la quantité desdits composés dans des compositions cosmétiques ou dermatologiques par rapport à la quantité normalement utilisée, en vue d'une efficacité accrue de ces derniers.

Ainsi, on peut utiliser, y compris dans les compositions destinées aux peaux et/ou aux muqueuses et/ou aux cuirs chevelus sensibles et/ou irritables et/ou allergiques, des agents susceptibles de provoquer une irritation de la peau tels que des actifs cosmétiques (ex : agent kératolytiques et/ou desquamant), des actifs dermatologiques (ex : rétinoïdes), certains tensioactifs, conservateurs, parfums, solvants, propulseurs, et leurs mélanges pour autant que lesdites compositions comprennent au moins un dérivé C-glycoside.

Au sens de l'invention, par « peau » on entend toute surface cutanée du corps incluant la peau et élargie au cuir chevelu et aux muqueuses et semi-muqueuses (telles que les lèvres).

Il a par ailleurs été mis en évidence que la substance P pouvait être responsable de manifestations cutanées caractérisant les peaux sensibles.

D'une manière générale, les peaux sensibles se définissent par une réactivité particulière de la peau.

Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultra-violets ou infrarouges. Il existe également des facteurs associés comme l'âge et le type de peau. Ainsi les peaux sensibles sont plus fréquentes parmi les peaux sèches ou grasses que parmi les peaux normales.

L'apparition de ces signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques. On entend par sensations dysesthésiques, des sensations plus ou moins douloureuses ressenties dans une zone cutanée comme les picotements, fourmillements, démangeaisons, brûlures, échauffements, inconforts, tiraillements, etc. Ces signes subjectifs existent le plus souvent en l'absence de signes cliniques visibles tels que la rougeur et les desquamations et en l'absence de réaction inflammatoire. On sait aujourd'hui que ces réactions d'irritation et d'intolérance cutanée sont notamment liées à une libération de neuropeptides par les terminaisons nerveuses de l'épiderme et du derme.

Par opposition aux peaux qualifiées d'allergiques, la réactivité d'une peau sensible ne relève pas d'un processus immunologique. Son mécanisme de réponse est dit "aspécifique". Elle est, à ce titre, à distinguer des peaux manifestant des réactions inflammatoires et allergiques de type dermatose, eczéma, et/ou ichtyose, et vis-à-vis desquelles un certain nombre de traitements ont déjà été proposés.

Pour des raisons évidentes, l'absence de signes visibles rend difficile le diagnostic de peau sensible. Le plus souvent ce diagnostic repose sur l'interrogatoire du patient. Cette symptomatologie a en outre pour intérêt de permettre de différencier la peau sensible, de l'irritation ou de l'allergie de contact pour lesquelles il existe en revanche des signes inflammatoires visibles.

En conséquence, la mise au point de produits "peaux sensibles" a nécessité de disposer d'outils d'évaluation de la réaction sensorielle de la peau. Les premiers outils se sont inspirés dès leur conception de la caractéristique essentielle des peaux sensibles à savoir la présence de signes d'inconfort induits par une application topique.

Ainsi, le "stinging test" à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelées "stingers" et considérés comme étant à peau sensible. En raison de cette sensibilité cutanée à l'application topique de produit, ces sujets sont alors sélectionnés pour tester des produits dits peaux sensibles.

Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine.

Ce second type de test, décrit dans la demande EP 1374 913, constitue également un autre moyen particulièrement utile pour le diagnostic de peaux sensibles.

Au sens de la présente invention, les peaux sensibles couvrent les peaux irritables et/ou allergiques et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable et/ou allergique est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température, l'humidité ou la laine.

Les cuirs chevelus "sensibles" ont une sémiologie clinique plus univoque : les sensations de démangeaisons et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

La demanderesse a mis en évidence le caractère antagoniste de substance P de dérivés C-glycoside. Ces composés présentent donc un intérêt pour une utilisation cosmétique comme agent pour traiter les signes des peaux sensibles.

La composition selon l'invention comprend un milieu physiologiquement acceptable, c'est-à-dire un milieu compatible avec toutes les matières kératiniques telles que la peau, le cuir chevelu, les ongles, les muqueuses, les yeux et les cheveux ou toute autre zone cutanée du corps. Cette composition peut être une composition cosmétique ou dermatologique et peut donc comprendre un milieu cosmétiquement ou pharmaceutiquement acceptable.

Relativement aux objets revendiqués, qu'il s'agisse de l'utilisation non thérapeutique, de l'utilisation à des fins thérapeutiques, du procédé cosmétique ou des compositions, le mode privilégié d'administration est la voie topique, à savoir l'application sur la peau.

### INGREDIENTS COSMETIQUE OU PHARMACEUTIQUE SUSCEPTIBLE DE PROVOQUER UNE IRRITATION DE LA PEAU

L'utilisation non thérapeutique selon la présente invention vise notamment à prévenir et/ou diminuer l'effet irritant d'une composition cosmétique ou dermatologique contenant ou plusieurs composés susceptibles de provoquer une irritation de la peau.

Une composition selon la présente invention comprend au moins un composé cosmétique ou pharmaceutique susceptible de provoquer une irritation de la peau.

Parmi ces composés on peut notamment citer des composés ou actifs cosmétiques, des composés ou actifs dermatologiques, des tensioactifs notamment des tensioactifs anioniques, des conservateurs, des détergents, des parfums et notamment des solutions alcooliques parfumantes, des solvants, des propulseurs et leurs mélanges.

Plus particulièrement à titre d'actifs dermatologiques ou cosmétiques on peut citer certains agents desquamants qui peuvent être également des agents de peeling.

Parmi les agents spécifiquement de peeling on peut citer des particules abrasives/exfoliantes de sources minérales, organiques, naturelles ou synthétiques. On peut plus particulièrement citer les particules de pierre ponce, de silice, des billes de polyéthylènes, de nylon et des poudres de noyaux de fruits.

Parmi ces agents desquamants les suivants sont susceptibles de provoquer une irritation de la peau : les acides monocarboxyliques saturés (acide acétique) et insaturés, les acides dicarboxyliques saturés et insaturés, les acides tricarboxyliques saturés et insaturés ; les α-hydroxyacides et β-hydroxyacides des acides monocarboxyliques ; les α-hydroxyacides et β-hydroxyacides des acides dicarboxyliques ; les α-hydroxyacides et β-hydroxyacides, des acides tricarboxiliques, les cétoacides, les α-cétoacides, les β-cétoacides d'acides polycarboxyliques, d'acides polyhydroxy monocarboxyliques, d'acides polyhydroxy bicarboxyliques et d'acides polyhydroxy tricarboxyliques.

Particulièrement parmi les α-hydroxyacides ou leurs esters on peut citer : les acides glycolique, dioïque comme l'acide octadécène dioïque ou Arlatone dioc DCA vendu par la société Uniqema, citrique, lactique, tartrique, malique ou mandélique, leur esters comme le tartrate de dialkyle (C12/C13) ou Cosmacol ETI, le citrate de tri-alcools C12-13 ramifiés ou Cosmacol ECI commercialisé par la société SASOL.

Parmi les β-hydroxyacides on peut citer : l'acide salicylique et ses dérivés (dont l'acide n-octanoyl 5-salicylique).

Parmi les α-cétoacides on peut citer l'acide ascorbique et ses dérivés.

Parmi les autres agents desquamants on peut citer : les acides pyruvique, gluconique, glucuronique, oxalique, malonique, succinique, acétique, gentisique, cinnamique, azélaique ; le phénol ; la résorcine ; l'urée et ses dérivés, l'hydroxyéthyl urée ou hydrovance® de chez NATIONAL STARCH ; les oligofucoses ; l'acide jasmonique et ses dérivés ; l'acide ascorbique et ses dérivés, l'acide trichloracétique ; l'extrait de Saphora japonica et le résvératrol.

Parmi les agents desquamants, ceux capables d'agir sur les enzymes impliqués dans la desquamation ou la dégradation des cornéodesmosomes peuvent également être susceptibles de provoquer une irritation de la peau.

Parmi ceux-ci, on peut notamment citer les agents chélatants des sels minéraux tels l'EDTA ; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyl glycine diacétate de sodium commercialisé par BASF sous la dénomination commerciale TRILON M^{®}) ; le miel ; les dérivés de sucre tels que l'O-octanoyl-6-D-maltose, le O-linoleyl-6-D-glucose et la N-acétyl glucosamine.

Les rétinoïdes sont également des composés susceptibles de provoquer une irritation de la peau. On peut par exemple citer parmi eux le rétinol et ses esters, le rétinal, l'acide rétinoïque et ses dérivés tels que ceux décrits dans les documents FR-A-2 570 377, EP-A-199 636, EP-A-325 540, EP-A-402 072, et l'adapalène.

Les sels et dérivés, comme les formes cis ou trans, les mélanges racémiques, les formes dextrogyres ou levogyres des composés cités précédemment sont aussi considérés comme des composés susceptibles de provoquer une irritation de la peau.

D'autres actifs dermatologiques ou cosmétiques susceptibles de provoquer une irritation de la peau sont également cités ci-après :
- l'urée et ces dérivés comme l'hydroxyéthyl urée ou hydrovance® de NATIONAL STARCH,
- certaines vitamines telles que la vitamine D et ses dérivés tels que la vitamine D3, la vitamine D2, le calcitriol, le calcipotriol, le tacalcitol, la 24,25-diOH vitamine D3, la 1-OH vitamine D2 et la 1,24-diOH vitamine D2 ; la vitamine B9 et ses dérivés,
- les peroxydes comme le peroxyde de benzoyle, l'eau oxygénée,
- les antichutes tels que le minoxidil et ses derivés tel que l'aminexyl,
- les teintures et les colorants capillaires comme les aminophénols et leurs dérivés tels que la para-phénylène diamine (p-PDA), la N-phenyl p-PDA, le toluène 2,5-diamine sulfate, la méta-phénylène diamine (m-PDA), la toluène 3,4-diamine et l'ortho-phénylène diamine (o-PDA),
- les agents anti-transpirants comme les sels d'Aluminium, tel que l'hydroxychlorure d'aluminium,
- les déodorants,
- les actifs dépilatoires et/ou de permanentes tels que les thioglycolates, l'ammoniaque,
- le thioglycolate et ses sels,
- le phénoxyéthanol,
- le 1,2-pentanediol,
- les solutions alcooliques parfumantes (parfums, eaux de toilette, après rasage, déodorants)
- les anthralines (dioxyanthranol),
- les anthranoïdes (par exemple ceux décrits dans le document EP-A- 319028),
- les sels de lithium,
- les dépigmentants (ex : hydroquinone, vitamine C à forte concentration, acide kojique),
- certains actifs amincissants à effet chauffant,
- les nicotinates et leurs dérivés,
- la capsaïcine,
- les actifs antipoux (pyréthrine),
- les antiprolifératifs tels que le 5-fluoro uracile ou le méthotrexate,
- les agents antiviraux,
- les antiparasitaires,
- les antifongiques,
- les antiprurigineux,
- les antiséborrhéiques,
- certaines filtres solaires,
- les propigmentants tels que les psoralènes et les méthylangécilines, et
- leurs mélanges.

Comme conservateurs, on peut citer le phénoxyéthanol, la chorehexidine et le chlorure de benzalkonium.

Comme tensioactifs, on peut citer les tensioactifs anioniques, cationiques et amphotères, plus particulièrement les tensioactifs anioniques tels que les alkyl sulfates et alkyl éther sulfates comme le lauryl sulfate et le lauryl éther sulfate, et leurs sels notamment de sodium.

Selon un mode de réalisation préféré de l'invention, le composé susceptible de provoquer une irritation de la peau est choisi parmi les rétinoïdes, les α-hydroxyacides, les β-hydroxyacides, les acides dicarboxyliques saturés et insaturés tels que l'acide octadécène dioique ou Arlatone DIOC DCA vendu par la société Uniqema, les tensioactifs anioniques, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique (HEPES), l'acide cinnamique et leurs mélanges.

Le composé susceptible de provoquer une irritation de la peau peut être présent dans la composition selon la présente invention dans une quantité suffisante pour provoquer une réaction d'irritation de la peau. A titre d'exemple, il peut être présent dans une teneur allant de 0,0001 à 70% en poids, de préférence de 0,01 à 50% en poids et mieux de 0,1 à 30% en poids par rapport au poids total de la composition.

### C-GLYCOSIDE

Un dérivé C-glycoside convenant à l'invention peut être un composé de formule générale (I) suivante : 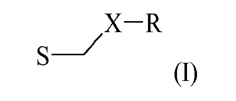 dans laquelle :
- R représente :
   - un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀, en particulier en C₃ à C₁₀ ;
   - un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀;
      la chaîne hydrocarbonée constituant lesdits radicaux pouvant, le cas échéant, être interrompue par 1, 2, 3 ou plus d'hétéroatomes choisis parmi :

   - un oxygène,
   - un soufre,
   - un azote, et
   - un silicium,
      et pouvant être éventuellement substituée par au moins un radical choisi parmi :

   - -OR₄,
   - -SR₄,
   - -NR₄R₅,
   - -COOR₄,
   - -CONHR₄,
   - -CN,
   - un atome d'halogène,
   - un radical hydrofluoro- ou perfluoro-alkyle, en C₁ à C₆, et/ou
   - un radical cycloalkyle en C₃ à C₈,
      avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀ , notamment en C₃ à C₁₂ ; ou un radical aryle en C₆ à C₁₀,
- X représente un radical choisi parmi les groupements : 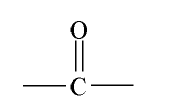 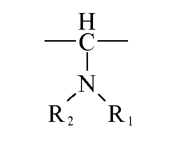 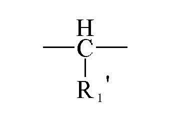 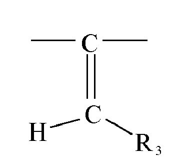 avec R₁, R₂ et R₃ représentant, indépendamment l'un de l'autre, un atome d'hydrogène, ou un radical R, avec R tel que défini précédemment, et R₁' représente un atome d'hydrogène, un groupe -OH ou un radical R tel que défini précédemment, R₁ pouvant désigner également un radical aryle en C₆ à C₁₀,
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β,
   ainsi que leurs sels cosmétiquement acceptables, leurs solvates tels que les hydrates et leurs isomères.

Dans le cadre de la présente invention, par "halogène", on entend le chlore, le fluor, le brome ou l'iode.

Le terme "aryle" désigne un cycle aromatique tel que phényle, éventuellement substitué par un ou plusieurs radicaux alkyles en C₁-C₄.

Le terme "cycloalkyle en C₃ à C₈" désigne un cycle aliphatique ayant de 3 à 8 atomes de carbone, incluant par exemple le cyclopropyle, le cyclopentyle et le cyclohéxyle.

Parmi les groupes alkyle convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Selon un mode de réalisation de l'invention, on peut utiliser un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine obligatoirement protégée, X et R conservant par ailleurs l'ensemble des définitions précédemment données.

Avantageusement, un monosaccharide de l'invention peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide de l'invention contenant jusqu'à 6 unités sucre peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-β-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le L-fucose, le D-galactose, le D-maltose et notamment le D-xylose.

Selon un autre mode de réalisation de l'invention, on peut utiliser des dérivés C-glycoside répondant à la formule (I) pour lesquels X représente un groupement choisi parmi -CO-, -CH(OH)-, -CH(NR₁R₂)-, -CH(R)-, en particulier -CO-, -CH(OH)-, -C(NH₂)-, -C(NHCH₂CH₂CH₂OH)-, -C(NHPh)-, -C(CH₃)-, et plus particulièrement un groupement - CO-, -CH(OH)-, -CH(NH₂)-, et préférentiellement un groupement -CH(OH)-, S et R conservant par ailleurs l'ensemble des définitions précédemment données.

Selon un autre mode de réalisation de l'invention, on peut utiliser un dérivé C-glycoside répondant à la formule (I) pour lesquels R représente un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀ , en particulier en C₃ à C₁₀, et éventuellement substitué comme décrit précédemment, S et X conservant par ailleurs l'ensemble des définitions précédemment données. De préférence, R désigne un radical linéaire en C₁-C₄, notamment C₁-C₃, éventuellement substitué par - OH, -COOH ou -COOR"₂, R"₂ étant un radical alkyle saturé en C₁-C₄, notamment éthyle. Préférentiellement R désigne un radical alkyle linéaire non substitué en C₁-C₄, notamment C₁-C₂, en particulier éthyle.

Parmi les dérivés C-glycoside de formule (I), on utilise de préférence ceux pour lesquels :
- R représente un radical alkyle en linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C_{20;} en particulier en C₃ à C₁₀, et éventuellement substitué comme décrit précédemment ;
- S représente un monosaccharide comme décrit précédemment ;
- X représente -CO-, -CH(OH)-, -CH(NR₁R₂)-, -CH(R)- comme décrit précédemment.

De préférence, on utilise un dérivé C-glycoside de formule (I) pour lesquels :
- R désigne un radical linéaire en C₁-C₄, notamment C₁-C₃, éventuellement subsitué par -OH, -COOH ou -COOR"₂, R"₂ étant un radical alkyle saturé en C₁-C₄, notamment éthyle ;
- S représente un monosaccharide comme décrit précédemment ;
- X représente un groupement choisi parmi -CO-, -CH(OH)-, -C(NH₂)-, -C(NHCH₂CH₂CH₂OH)-, -C(NHPh)-, -C(CH₃)-, et plus particulièrement un groupement -CO-, -CH(OH)-, -CH(NH₂)-, et préférentiellement un groupement -CH(OH)-.

Préférentiellement, on utilise un dérivé C-glycoside de formule (I) pour lesquels :
- R désigne un radical alkyle linéaire non substitué en C₁-C₄, notamment C₁-C₂, en particulier éthyle;
- S représente un monosaccharide comme décrit précédemment ; notamment le D-glucose, le D-xylose, la N-acétyl-D-glucosamine ou le L-fucose, et en particulier le D-xylose;
- X représente un groupement choisi parmi -CO-, -CH(OH)-, -CH(NH₂)-, et préférentiellement un groupement -CH(OH)-.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycoside de formule (I), utilisés selon l'invention, on considère tout particulièrement :
1. C-β-D-xylopyranoside-n-propane-2-one ;
2. C-α-D-xylopyranoside-n-propane-2-one ;
3. 1-[2-(3-hydroxy-propylamino)-propyl]-C-β-D-xylopyranose ;
4. 1-[2-(3-hydroxy-propylamino)-propyl]-C-α-D-xylopyranose ;
5. C-β-D-xylopyranoside-2-hydroxy-propane ;
6. C-α-D-xylopyranoside-2-hydroxy-propane ;
7. C-β-D-xylopyranoside-2-amino-propane ;
8. C-α-D-xylopyranoside-2-amino-propane ;
9. C-β-D-xylopyranoside-2-phénylamino-propane ;
10. C-α-D-xylopyranoside-2-phénylamino-propane ;
11. ester éthylique de l'acide 3-méthyl-4-(C-β-D-xylopyranoside)-butyrique ;
12. ester éthylique de l'acide 3-méthyl-4-(C-α-D-xylopyranoside)-butyrique ;
13. acide 6-(C-β-D-xylopyranoside)-5-céto-hexanoique ;
14. acide 6-(C-α-D-xylopyranoside)-5-céto-hexanoique ;
15. acide 6-(C-β-D-xylopyranoside)-5-hydroxy-hexanoique ;
16. acide 6-(C-α-D-xylopyranoside)-5-hydroxy-hexanoique ;
17. acide 6-(C-β-D-xylopyranoside)-5-amino-hexanoique ;
18. acide 6-(C-α-D-xylopyranoside)-5-amino-hexanoique ;
19. acide 6-(C-β-D-xylopyranoside)-5-phenylamino-hexanoique ;
20. acide 6-(C-α-D-xylopyranoside)-5-phenylamino-hexanoique ;
21. 1-(C-β-D-xylopyranoside)-hexane-2,6-diol,
22. 1-(C-α-D-xylopyranoside)-hexane-2,6-diol ;
23. acide 5-(C-β-D-xylopyranoside)-4-céto-pentanoique ;
24. acide 5-(C-α-D-xylopyranoside)-4-céto-pentanoique ;
25. acide 5-(C-β-D-xylopyranoside )-4-hydroxy-pentanoique ;
26. acide 5-(C-α-D-xylopyranoside )-4-hydroxy-pentanoique ;
27. acide 5-(C-β-D-xylopyranoside)-4-amino-pentanoique ;
28. acide 5-(C-α-D-xylopyranoside)-4-amino-pentanoique ;
29. acide 5-(C-β-D-xylopyranoside)-4-phénylammo-pentanoique ;
30. acide 5-(C-α-D-xylopyranoside)-4-phénylamino-pentanoique ;
31. 1-(C-β-D-xylopyranoside)-pentane-2,5-diol ;
32. 1-(C-α-D-xylopyranoside)-pentane-2,5-diol ;
33. 1-(C-β-D-fucopyranoside)-propane-2-one,
34. 1-(C-α-D-fucopyranoside)-propane-2-one ;
35. 1-(C-β-L-fucopyranoside)-propane-2-one,
36. 1-(C-α-L-fucopyranoside)-propane-2-one ;
37. 1-(C-β-D-fucopyranoside)-2-hydroxy-propane,
38. 1-(C-α-D-fucopyranoside)-2-hydroxy-propane ;
39. 1-(C-β-L-fucopyranoside)-2-hydroxy-propane,
40. 1-(C-α-L-fucopyranoside)-2-hydroxy-propane ;
41. 1-(C-β-D-fucopyranoside)-2-amino-propane ;
42. 1-(C-α-D-fucopyranoside)-2-amino-propane ;
43. 1-(C-β-L-fucopyranoside)-2-amino-propane ;
44. 1-(C-α-L-fucopyranoside)-2-amino-propane ;
45. 1-(C-β-D-fucopyranoside)-2-phénylamino-propane ;
46. 1-(C-α-D-fucopyranoside)-2-phénylamino-propane ;
47. 1-(C-β-L-fucopyranoside)-2-phénylamino-propane ;
48. 1-(C-α-L-fucopyranoside)-2-phénylamino-propane ;
49. ester éthylique de l'acide 3-méthyl-4-(C-β-D-fucopyranoside)-butyrique ;
50. ester éthylique de l'acide 3-méthyl-4-(C-α-D-fucopyranoside)-butyrique ;
51. ester éthylique de l'acide 3-méthyl-4-(C-β-L-fucopyranoside)-butyrique ;
52. ester éthylique de l'acide 3-méthyl-4-(C-α-L-fucopyranoside)-butyrique ;
53. acide 6-(C-β-D-fucopyranoside)-5-céto-hexanoique ;
54. acide 6-(C-α-D-fucopyranoside)-5-céto-hexanoique ;
55. acide 6-(C-β-L-fucopyranoside)-5-céto-hexanoique ;
56. acide 6-(C-α-L-fucopyranoside)-5-céto-hexanoique ;
57. acide 6-(C-β-D-fucopyranoside)-5-hydroxy-hexanoique ;
58. acide 6-(C-α-D-fucopyranoside)-5-hydroxy-hexanoique ;
59. acide 6-(C-β-L-fncopyranoside)-5-hydroxy-hexanoique ;
60. acide 6-(C-α-L-fucopyranoside)-5-hydroxy-hexanoique ;
61. acide 6-(C-β-D-fncopyranoside)-5-ammo-hexanoique ;
62. acide 6-(C-α-D-fucopyranoside)-5-amino-hexanoique ;
63. acide 6-(C-β-L-fucopyranoside)-5-amino-hexanoique ;
64. acide 6-(C-α-L-fucopyranoside)-5-amino-hexanoique ;
65. 1-(C-β-D-fucopyranoside)-hexane-2,6-diol,
66. 1-(C-α-D-fucopyranoside)-hexane-2,6-diol ;
67. 1-(C-β-L-fucopyranoside)-hexane-2,6-diol ;
68. 1-(C-α-L-fucopyranoside)-hexane-2,6-diol ;
69. acide 5-(C-β-D-fucopyranoside)-4-céto-pentanoique ;
70. acide 5-(C-α-D-fucopyranoside)-4-céto-pentanoique ;
71. acide 5-(C-β-L-fucopyranoside)-céto-pentanoique ;
72. acide 5-(C-α-L-fucopyranoside)-4-céto-pentanoique ;
73. acide 5-(C-β-D-fucopyranoside)-4-hydroxy-pentanoique ;
74. acide 5-(C-α-D-fucopyranoside)-4-hydroxy-pentanoique ;
75. acide 5-(C-β-L-fucopyranoside)-4-hydroxy-pentanoique;
76. acide 5-(C-α-L-fucopyranoside)-4-hydroxy-pentanoique ;
77. acide 5-(C-β-D-fucopyranoside)-4-amino-pentanoique;
78. acide 5-(C-α-D-fucopyranoside)-4-amino-pentanoique
79. acide 5-(C-β-L-fucopyranoside)-4-amino-pentanoique ;
80. acide 5-(C-α-L-fucopyranoside)-4-amino-pentanoique ;
81. 1-(C-β-D-fucopyranoside)-pentane-2,5-diol,
82. 1-(C-α-D-fucopyranoside)-pentane-2,5-diol ;
83. 1-(C-β-L-fucopyranoside)-pentane-2,5-diol ;
84. 1-(C-α-L-fucopyranoside)-pentane-2,5-diol ;
85. 1-(C-β-D-glucopyranosyl)-2-hydroxy-propane ;
86. 1-(C-α-D-glucopyranosyl)-2-hydroxy-propane ;
87. 1-(C-β-D-glucopyranosyl)-2-amino-propane ;
88. 1-(C-α-D-glucopyranosyl)-2-amino-propane ;
89. 1-(C-β-D-glucopyranosyl)-2-phénylamino-propane ;
90. 1-(C-α-D-glucopyranosyl)-2-phénylamino-propane ;
91. ester éthylique de l'acide 3-méthyl-4-(C-β-D-glucopyranosyl)-butyrique ;
92. ester éthylique de l'acide 3-méthyl-4-(C-α-D-glucopyranosyl)-butyrique ;
93. acide 6-(C-β-D-glucopyranosyl)-5-céto-hexanoique ;
94. acide 6-(C-α-D-glucopyranosyl)-5-céto-hexanoique ;
95. acide 6-(C-β-D-glucopyranosyl)-5-hydroxy-hexanoique ;
96. acide 6-(C-α-D-glucopyranosyl)-5-hydro xy-hexanoique ;
97. acide 6-(C-β-D-glucopyranosyl)-5-amino-hexanoique ;
98. acide 6-(C-α-D-glucopyranosyl)-5-amino-hexanoique ;
99. acide 6-(C-β-D-glucopyranosyl)-5-phénylamino-hexanoique ;
100. acide 6-(C-α-D-glucopyranosyl)-5-phénylamino-hexanoique ;
101. 1-(C-β-D-glucopyranosyl)-hexane-2,6-diol ;
102. 1-(C-α-D-glucopyranosyl)-hexane-2,6-diol ;
103. acide 6-(C-β-D-glucopyranosyl)-5-céto-pentanoique ;
104. acide 6-(C-α-D-glucopyranosyl)-5-céto-pentanoique ;
105. acide 6-(C-β-D-glucopyranosyl)-5-hydroxy-pentanoique ;
106. acide 6-(C-α-D-glucopyranosyl)-5-hydroxy-pentanoique ;
107. acide 6-(C-β-D-glucopyranosyl)-5-ammo-pentanoique ;
108. acide 6-(C-α-D-glucopyranosyl)-5-hydroxy-pentanoique ;
109. acide 6-(C-β-D-glucopyranosyl)-5-phénylamino-pentanoique ;
110. acide 6-(C-α-D-glucopyranosyl)-5-phénylamino-pentanoique ;
111. 1-(C-β-D-glucopyranosyl)-pentane-2,5-diol ;
112. 1-(C-α-D-glucopyranosyl)-pentane-2,5-diol,
113. 1-(C-β-D-galactopyranosyl)-2-hydroxy-propane ;
114. 1-(C-α-D-galactopyranosyl)-2-hydroxy-propane ;
115. 1-(C-β-D-galactopyranosyl)-2-amino-propane ;
116. 1-(C-α-D-galactopyranosyl)-2-amino-propane ;
117. 1-(C-β-D-galactopyranosyl)-2-phénylamino-propane ;
118. 1-(C-α-D-galactopyranosyl)-2-phénylamino-propane ;
119. ester éthylique de l'acide 3-méthyl-4-(β-D-galactopyranosyl)-butyrique ;
120. ester éthylique de l'acide 3-méthyl-4-(α-D-galactopyranosyl)-butyrique ;
121. acide 6-(C-β-D-galactopyranosyl)-5-céto-hexanoique ;
122. acide 6-(C-α-D-galactopyranosyl)-5-céto-hexanoique ;
123. acide 6-(C-β-D-galactopyranosyl)-5-hydroxy-hexanoique ;
124. acide 6-(C-α-D-galactopyranosyl)-5-hydroxy-hexanoique ;
125. acide 6-(C-β-D-galactopyranosyl)-5-ammo-hexanoique ;
126. acide 6-(C-α-D-galactopyranosyl)-5-amino-hexanoique ;
127. acide 6-(C-β-D-galactopyranosyl)5-phénylamino-hexanoique ;
128. acide 6-(C-α-D-galactopyranosyl)5-phénylamino-hexanoique ;
129. 1-(C-β-D-galactopyranosyl)-hexane-2,6-diol,
130. 1-(C-α-D-galactopyranosyl)-hexane-2,6-diol ;
131. acide 6-(C-β-D-galactopyranosyl)-5-céto-pentanoique ;
132. acide 6-(C-α-D-galactopyranosyl)-5-céto-pentanoique ;
133. acide 6-(C-β-D-galactopyranosyl)-5-hydroxy-pentanoique ;
134. acide 6-(C-α-D-galactopyranosyl)-5-hydroxy-pentanoique ;
135. acide 6-(C-β-D-galactopyranosyl)-5-amino-pentanoique ;
136. acide 6-(C-α-D-galactopyranosyl)-5-amino-pentanoique ;
137. acide 6-(C-β-D-galactopyranosyl)-5-phénylamino-pentanoique ;
138. acide 6-(C-α-D-galactopyranosyl)-5-phénylamino-pentanoique ;
139. 1-(C-β-D-galactopyranosyl)-pentane-2,6-diol ;
140. 1-(C-α-D-galactopyranosyl)-pentane-2,6-diol ;
141. 1-(C-β-D-fncofnranosyl)-propane-2-one ;
142. 1-(C-α-D-fucofuranosyl)-propane-2-one ;
143. 1-(C-β-L-fucofuranosyl)-propane-2-one ;
144. 1-(C-α-L-fucofuranosyl)-propane-2-one ;
145. 3'-(acétamido-C-β-D-glucopyranosyl)-propane-2'-one ,
146. 3'-(acétamido-C-α-D-glucopyranosyl)-propane-2'-one ;
147. 1-(acétamido-C-β-D-glucopyranosyl)-2-hydroxyl-propane ;
148. 1-(acétamido-C-β-D-glucopyranosyl)-2-amino-propane ;
149. 1-(acétamido-C-β-D-glucopyranosyl)-2-phénylamino-propane ;
150. 1-(acétamido-C-α-D-glucopyranosyl)-2-phénylamino-propane ;
151. ester éthylique de l'acide 3-méthyl-4-(acétamido-C-β-D-glucopyranosyl)-butyrique;
152. ester éthylique de l'acide 3-méthyl-4-(acétamido-C-α-D-glucopyranosyl)-butyrique;
153. acide 6-(acétamido-C-β-D-glucopyranosyl)-5-céto-hexanoique;
154. acide 6-(acétamido-C-α-D-glucopyranosyl)-5-céto-hexanoique ;
155. acide 6-(acétamido-C-β-D-glucopyranosyl)-5-hydroxy-hexanoique;
156. acide 6-(acétamido-C-α-D-glucopyranosyl)-5-hydroxy-hexanoique ;
157. acide 6-(acétamido-C-β-D-glucopyranosyl)-5-amino-hexanoique ;
158. acide 6-(acétamido-C-α-D-glucopyranosyl)-5-amino-hexanoique ;
159. acide 6-(acétamido-C-β-D-glucopyranosyl)-5-phénylamino-hexanoique ;
160. acide 6-(acétamido-C-α-D-glucopyranosyl)-5-phénylamino-hexanoique ;
161. 1-(acétamido-C-β-D-glucopyranosyl)-hexane-2,6-diol ;
162. 1-(acétamido-C-α-D-glucopyranosyl)-hexane-2,6-diol ;
163. acide 6-(acétamido-C-β-D-glucopyranosyl)-5-céto-pentanoique;
164. acide 6-(acétamido-C-α-D-glucopyranosyl)-5-céto-pentanoique ;
165. acide 6-(acétamido-C-β-D-glucopyranosyl)-5-hydroxy- pentanoique ;
166. acide 6-(acétamido-C-α-D-glucopyranosyl)-5-hydroxy- pentanoique ;
167. acide 6-(acétamido-C-β-D-glucopyranosyl)-5-amino- pentanoique ;
168. acide 6-(acétamido-C-α-D-glucopyranosyl)-5-amino- pentanoique ;
169. acide 6-(acétamido-C-β-D-glucopyranosyl)-5-phénylamino- pentanoique ;
170. acide 6-(acétamido-C-α-D-glucopyranosyl)-5-phénylamino- pentanoique ;
171. 1-(acétamido-C-β-D-glucopyranosyl)-pentane-2,5-diol ;
172. 1-(acétamido-C-α-D-glucopyranosyl)-pentane-2,5-diol.

A titre illustratif et non limitatif des dérivés C-glycoside convenant plus particulièrement à l'invention, on peut notamment citer les dérivés suivants :
- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1-(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside) -2-hydroxy-propane,
- le 1-(C-β-D-Glucopyranosyl)-2-hydroxyl-propane,
- le 1-(C-α-D-Glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucokranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.

Selon un mode de réalisation particulier, le dérivé C-glycoside est le C-β-D-xylopyranoside-2-hydroxy-propane sous forme de solution à 30% en poids en matière active dans un mélange eau/propylèneglycol (60/40 % en poids) tel que le produit fabriqué par CHIMEX sous la dénomination commerciale « MEXORYL SBB ^{®}».

Bien entendu, selon l'invention, un dérivé C-glycoside répondant à la formule (I) peut être utilisé seul ou en mélange avec d'autres dérivés C-glycosides et en toute proportion.

Un dérivé C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre un dérivé C-glycoside à raison d'environ 0,0001 % à environ 25 % en poids en matière active par rapport au poids total de la composition, et en particulier d'environ 0,001 % à environ 10 % en poids en matière active, et plus particulièrement d'environ 0,05 % à environ 5 % en poids en matière active de dérivé C-glycoside par rapport au poids total de la composition.

Le rapport massique entre le composé cosmétique ou pharmaceutique susceptible de provoquer une irritation de la peau et le dérivé C-glycoside peut varier de 0,000004 à 70 000, voire de 0,001 à 5 000 et de préférence de 0,002 à 2 000.

### ACTIFS ADDITIONNELS

Le dérivé C-glycoside utilisé selon l'invention peut également être associé à au moins 0,00001 % à 95 % en poids d'un agent anti-inflammatoire, un autre agent apaisant ou leur mélange.
Comme exemples « d'agents anti-inflammatoires », on peut citer :
- un antagoniste de cytokines inflammatoires ;
- un anti-inflammatoire stéroïdien (Hydrocortisone, betaméthasone, dexaméthasone etc..) ;
- un anti-inflammatoire non stéroïdien comme l'aspirine ou le paracetamol ; et leurs mélanges.

Par « antagonistes de cytokines inflammatoires » selon l'invention, on entend un composé susceptible d'inhiber la synthèse et/ou la libération d'une ou plusieurs cytokines inflammatoires. Entrent également dans la définition d'un antagoniste des cytokines inflammatoires, les composés qui inhibent ou bloquent la fixation de ces cytokines sur leur(s) récepteur(s).

On peut citer par exemple les composés antagonistes de l'IL-1, de l'IL-8, du TNFα et du TNFβ, le tripeptide Lys-Pro-Val (KPV), et tous les dérivés de l'αMSH et peptidométiques apparentés caractérisés par une activité de type αMSH par liaison sur le récepteur ou par le contrôle de la libération d'IL1, d'IL8 ou encore de TNFα, tous les inhibiteurs de libération des cytokines (classe thérapeutique des CSAIDs pour : cytokine suppressive anti-inflammatory drugs), la famille des pyrimidine N-Oxyde substitués (EP99401719.2 (priorité FR9809509) et EP 99402771.2 (priorité FR9814211) inducteurs de lipoxine A4, les extraits d'algues capable de moduler la production des cytokines par les kératinocytes comme le Phycosaccharide® commercialisé par la société CODIF, extrait hydroglycolique de l'algue *Laminaria saccharina,* notamment la Phlorogine®, commercialisée par la société SECMA, les extraits *d'Aloe vera* ou de *Gingko biloba ;* l'antagoniste naturel à l'IL-1 (IL-1RA) et l'extrait d'écorces et de racines de *terminalia sericea* ou SERICOSIDE 3058500 commercialisés par la société INDENA.

Selon un mode de réalisation particulier de l'invention, l'agent anti-inflammatoire est choisi parmi les extraits d'algues capable de moduler la production des cytokines par les kératinocytes comme le Phycosaccharide® commercialisé par la société CODIF, l'extrait hydroglycolique de l'algue *Laminaria saccharina,* notamment la Phlorogine® commercialisée par la société SECMA, les extraits *d'Aloe vera,* l'extrait d'écorces et de racines de *terminalia sericea* ou SERICOSIDE 3058500 commercialisés par la société INDENA.

Les agents anti-inflammatoires sont de préférence présents dans les compositions conformes à l'invention à une concentration pouvant varier entre 0,00001 et 10 % en poids environ par rapport au poids total de la composition. Encore plus préférentiellement, la concentration en composé anti-inflammatoire peut varier entre 0,0005% et 2 % en poids par rapport au poids total de la composition.

Comme exemples « d'agents apaisants » utilisables dans les compositions de l'invention, on peut citer:
- l'allantoïne ;
- l'acide bêta-glycyrrhétinique, les extraits en contenant comme par exemple l'extrait de Glycyrrhiza Glabra (réglisse) et les complexes en contenant comme le complexe allantoïne/acide glycyrrhétinique ;
- les planctons, lyophilisés ou non, leurs extraits et leurs complexes ;
- l'escine et les extraits végétaux en contenant comme l'extrait de marron d'inde ;
- les dérivés de xanthine comme le chlorhydrate de di-éthylaminoéthyl théophylline ;
- les eaux et extraits (par exemple aqueux, hydroalcooliques ou hydroglycoliques) de fleurs et de plantes, comme l'eau de bleuet, l'eau de camomille, l'eau de menthe, l'eau de tilleul, l'eau de rose, les extraits de Rosacées (ex : *Rosa gallica),* les extraits de pivoine, les extraits d'aubépine, les extraits de millefeuille, les extraits de mauve, les extraits de souci, les extraits de melilot, les extraits de sauge, l'eau de sureau, les extraits de ginkgo biloba, les extraits d'arnica, les extraits d'origan, les extraits de thé vert, les extraits de fleurs de nénuphar, les extraits d'Iris, les extraits d'écorce de bouleau, les extraits d'Aloe vera;
- l'acide asiatique et les extraits végétaux en contenant comme la Centella Asiatica ;
- le bisabolol ;
- les extraits de fruits, comme l'extrait d'ananas, l'extrait de papaye; de goyave;
- les algues notamment du type *Laminaria* (par exemple rouges ou brunes) tel que l'extrait d'algue brune *Padina Pavonica* comme HPS 3 PADINA PAVONICA commercialisé par la société Alban Muller ;
- les pyrrolidone carboxylates et notamment de zinc (Zn-PCA) ou de cuivre (Cu-PCA) ;
- les huiles d'origine végétale, comme l'huile de graine de canola et l'huile de karité ;
- les huiles essentielles, par exemple de coriandre, de mélisse, de lavande, de menthe, de camomille et leurs mélanges ;
- l'acide acexamique et l'acide transexamique (acide trans-4, aminométhylcyclohexane carboxylique) ;
- l'acide ursolique et les extraits en contenant comme l'extrait de feuille de romarin ;
- les polysaccharides contenant du fucose, comme le FUCOGEL 1000, vendu par Solabia (solution aqueuse comprenant 1% de matière sèche de polysaccharide comprenant du fucose, du galactose et de l'acide galacturonique) ;
- les électrolytes et en particulier un mélange aqueux comprenant de 30 à 35 % du chlorure de magnésium, de 20 à 28 % de chlorure de potassium, de 3 à 10 % de chlorure de sodium, de 0,2 à 1 % de chlorure de calcium, de 0,1 à 0,6 % de bromure de magnésium et de 0,1 à 0,5 % d'insolubles, le dit mélange étant ici appelé « mélange des sels de la mer Morte » (« Dead Sea Bath Salts ») car il correspond aux principaux sels contenus dans la mer Morte;
- les galactolipides par exemple issus d'avoine, tels que par exemple le digalactosyl diglycéride ou le monogalactosyl diglycéride ;
- les acides aminés, leurs dérivés et leurs sels, tels que le sel de sodium d'aminoacides greffés sur des chaînes cocoyle, commercialisé sous forme d'un mélange sous la dénomination SEPICALM S par la société SEPPIC, le capryloylglycine commercialisé sous la dénomination LIPACIDE C8G par la société SEPPIC et le mélange de capryloylglycine, de cannelle et de sarcosine commercialisé sous la dénomination SEPICONTROL A5 par la société SEPPIC ;
- les antagonistes de TNF-alpha tels que la lisophylline, l'A802715, la sulfasalazine, le CDP-571 (anticorps anti-TNF-alpha), le MDL-201112 ;
- les antagonistes de substance P tels que le sendide, le spantide II, et les peptides décrits dans la demande EP-A-680749, les extraits de bactérie filamenteuse décrits dans la demande EP-A-761204 ;
- les antagonistes de CGRP, tels que le CGRP 8-37, les anticorps anti-CGRP, ou des extraits végétaux à activité antagoniste du CGRP *(ex : Iris pallida).*
- les sels divalents de strontium, de zinc, de manganèse, de magnésium, de calcium, tels que ceux décrits dans les documents WO-A-96/19184, WO-A-96/19182 et WO-A-96/19228 ;
et leurs mélanges.

En particulier, l'autre agent apaisant pourra avantageusement être choisi parmi l'allantoine, l'acide bêta-glycyrrhétinique, les extraits en contenant comme par exemple l'extrait de Glycyrrhiza Glabra (réglisse) et les complexes en contenant comme le complexe allantoïne/acide glycyrrhétinique ; les planctons, lyophilisés ou non, leurs extraits et leurs complexes ; les eaux et extraits de fleurs et de plantes : eaux de camomille, de tilleul, rose, extraits de bouleau ; le bisabolol ; les huiles essentielles par exemple de coriandre ; les algues notamment du type *Laminaria* (par exemple rouges ou brunes) tel que l'extrait d'algue brune *Padina Pavonica* comme HPS 3 PADINA PAVONICA commercialisé par la société Alban Muller ; l'acide acexamique et l'acide transexamique (acide trans-4, aminométhylcyclohexane carboxylique) ; l'acide ursolique et les extraits en contenant comme l'extrait de feuille de romarin ; les polysaccharides contenant du fucose, comme le FUCOGEL 1000, vendu par la société Solabia ; les électrolytes et en particulier un mélange aqueux comme le « mélange des sels de la mer Morte » (« Dead Sea Bath Salts ») ; les acides aminés comme les Sepicalm S et VG de Seppic et les sels divalents de magnésium tel que le magnésium gluconate.

L'invention a encore pour objet une composition pour application topique sur la peau et/ou les muqueuses et/ou le cuir chevelu comprenant dans un milieu physiologiquement acceptable :
- 0,00001 % à 95 % en poids d'au moins un composé anti-inflammatoire et/ou apaisant et,
- au moins un dérivé C-glycoside.

Les composés anti-inflammatoire et/ou apaisant sont tels que définis ci-dessus.

Selon un mode de réalisation particulier, la présente invention concerne une composition pour application topique sur la peau et/ou les muqueuses et/ou le cuir chevelu comprenant, dans un milieu physiologiquement acceptable, au moins un dérivé C-glycoside et au moins un extrait de rose, en particulier du genre Rosa.

Selon ce mode de réalisation particulier, le dérivé C-glycoside est tel que défini ci-dessus, y compris dans ses définitions préférées et notamment est le C-β-D-xylopyranoside-2-hydroxy-propane, notamment sous forme de solution à 30% en poids en matière active dans un mélange eau/propylèneglycol (60/40 % en poids) tel que le produit fabriqué par CHIMEX sous la dénomination commerciale « MEXORYL SBB ^{®}».

Le genre Rosa comporte plus de 1000 espèces parmi lesquelles on peut citer *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa ou encore Rosa villosa.*

L'extrait de végétal du genre Rosa utilisé selon l'invention peut être un extrait préparé à partir de matériel issu d'au moins un végétal appartenant à une espèce choisie parmi *Rosa alba L., Rosa alpina, Rosa canina L., Rosa centifolia L., Rosa cinnamonea, Rosa damascena Mill., Rosa gallica L., Rosa repens, Rosa rubrifolia, Rosa rubiginosa, Rosa sempervirens, Rosa spinosissima, Rosa stylosa, Rosa tomentosa, Rosa villosa.*

Préférentiellement selon l'invention le végétal appartient à l'espèce *Rosa gallica L..*

L'extrait d'au moins un végétal du genre Rosa utilisé selon l'invention peut être obtenu à partir de matériel végétal issu de plante entière ou de partie de plante comme les feuilles, les tiges, les fleurs, les pétales, les racines ou encore des cellules indifférenciées.

Selon l'invention, la quantité en extrait du végétal du genre Rosa utilisée dans la composition est bien entendu fonction de l'effet recherché et peut donc varier dans une large mesure.

Toute méthode d'extraction connue de l'homme du métier peut être utilisée pour préparer l'extrait contenu dans la composition selon l'invention.

On peut en particulier citer les extraits aqueux, alcooliques ou utilisant un solvant organique.

Par solvant aqueux on entend tout solvant constitué totalement ou pour part d'eau. On peut citer ainsi l'eau elle-même, les solvant alcooliques en toute proportion ou encore les solvants constitués d'eau et d'un composé comme le propylène glycol en toute proportion. Parmi les solvants alcooliques on peut citer notamment l'éthanol.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélangée dans un solvant approprié avant utilisation.

L'extrait végétal du genre Rosa décrit précédemment utilisé selon l'invention peut être présent dans la composition, en une quantité allant de 0,001 à 50 % en poids, par rapport au poids total de la composition, de préférence allant de 0,01 % à 30 % en poids, et préférentiellement allant de 0,1 à 15 % en poids.

### COMPOSITION

La composition de l'invention peut être une composition cosmétique ou dermatologique.

Le dérivé C-glycoside selon l'invention pourra être incorporé dans la composition cosmétique ou dermatologique qui contient un composé susceptible de provoquer une irritation de la peau ou, selon une alternative, être incorporé dans une composition apaisante distincte, qui sera appliquée après et/ou avant la composition contenant le composé susceptible de provoquer une irritation de la peau suivant si on désire enclencher la phase inflammatoire qui peut pour certaines applications utile (vasodilatation, dépilation etc...) au contraire l'éviter.

Dans le cas de certains agents desquamants ou agents de peeling par exemple, il pourra en effet être avantageux d'appliquer dans un premier temps la composition contenant ledit agent de peeling destiné à induire des remaniements pro-inflammatoires acellulaires cutanés (vasodilatation) pour remodeler ou dépiler l'épiderme.

Et dans un deuxième temps, appliquer une composition apaisante contenant un dérivé C-glycoside, ledit dérivé C-glycoside étant destiné à empêcher que les événements pro-inflammatoires acellulaires ne soient relayés par une étape de mobilisation et de chimioattraction cellulaire, pouvant conduire à une réaction inflammatoire aiguë et une phase de chronicité indésirable mettant en oeuvre des mécanismes immunologiques pouvant être délétères lorsqu'ils se retournent contre l'organisme.

Le milieu physiologiquement acceptable peut comprendre de l'eau, des solvants organiques tels qu'un alcool en C₁-C₈, notamment l'éthanol, l'isopropanol, le tert-butanol, le n-butanol; un polyol tel que la glycérine ; un glycol comme le butylène glycol, l'isoprène glycol, le propylène glycol, les polyéthylène glycols tels que le PEG-8 ; les éthers de polyol, l'acétone, l'acétate d'éthyle.

La composition de l'invention peut ainsi constituer une composition de traitement ou de soin de la peau (y compris le cuir chevelu), des fibres kératiniques (cheveux, cils, sourcils), des ongles ou des lèvres, ou une composition de protection solaire ou de bronzage artificiel, ou encore un produit nettoyant ou démaquillant de la peau, des cheveux, des sourcils ou des cils, un produit déodorant ou encore un composé parfumant. Elle est alors généralement non colorée ou faiblement colorée, et elle peut contenir éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), comme crème de soin de jour ou de nuit pour la peau du visage et/ou du corps. Elle peut, en outre, se présenter sous forme de shampooing traitant ou non, colorant ou non, et de composé après shampooing.

La composition selon l'invention peut également constituer une composition cosmétique colorée et notamment une composition de maquillage de la peau, des fibres kératiniques (cheveux ou cils) et/ou des muqueuses, en particulier un fond de teint, un blush, un fard à joues ou à paupières, un mascara, un eye-liner, un composé anti-cernes en stick, un vernis à ongle, un rouge à lèvres ou un brillant à lèvres, présentant éventuellement des propriétés de soin ou de traitement, un tatouage corps.

Ainsi, l'invention a encore pour objet l'utilisation cosmétique de la composition telle que définie ci-dessus, pour le soin, le traitement, le nettoyage et/ou le maquillage des matières kératiniques (peau y compris le cuir chevelu, fibres kératiniques et muqueuses).

La composition selon l'invention peut se présenter sous les formes galéniques classiquement utilisées pour une application topique et notamment sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de nanoémulsions, de dispersions vésiculaires de type ionique et/ou non ionique, ou des dispersions cire/phase aqueuse. Ces compositions sont préparées selon les méthodes usuelles.

Elle peut également se présenter sous la forme d'un système transdermique permettant une libération active ou passive du(des) actif(s) par transdermie, par exemple de type patch ou gel patch (hydrogel).

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique.

L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

On pourra également utiliser des agents hydratants parmi lesquels on peut notamment citer :
- la glycérine,
- l'hydroxyéthyl urée ou hydrovance® de NATIONAL STARCH,
- les polyols,
- les acides aminés et leurs dérivés N-acylés,
- l'acide pyrrolidone carboxylique et ses sels et dérivés N-acylés,
- les sucres, leurs alkyléthers et leurs alkylesters,
- les polyholosides hétérogènes,
- le tréhalose,
- l'ectoïne,
- les glycosaminoglycannes et leurs sulfates,
- les phospholipides et les polymères contenant des groupes phosphorylcholine,
- le cholestérol,
- les phytostérols, et
- l'acide hyaluronique.

Comme huiles, on utilisera par exemple des huiles hydrocarbonées d'origine végétale, les esters et les éthers de synthèse, les huiles de silicone et leurs mélanges.

On pourra également ajouter des agents tensioactifs pour homogénéiser le mélange des phases aqueuse et huileuse.

Les exemples suivants sont présents à titre illustratif et non limitatif du domaine de l'invention.

Le dérivé C-glycoside utilisé est le C-β-D-xylopyranoside-2-hydroxy-propane fabriqué par la société CHIMEX sous la dénomination commerciale « MEXORYL SBB ® ». Il se présente sous la forme d'une solution à 30 % en poids en matière active dans un mélange eau/propylèneglycol 60/40.

### EXEMPLES

### Exemple 1 : Evaluation de l'effet anti-irritant d'un composé C-glycoside

Le test consiste à évaluer l'effet du C-β-D-xylopyranoside-2-hydroxy-propane sur l'inhibition de la réponse des cellules épidermiques superficielles à un agent susceptible de provoquer une irritation de la peau, le rétinol, par dosage de l'interleukine-8 secrétée par des kératinocytes épidermiques humains normaux (NHEK) après stimulation avec l'agent susceptible de provoquer une irritation de la peau.

### - Principe et but de l'étude :

Cette étude utilise le test mis au point par Wilmer [Wilmer, J. L. et al., J. invest. Dermatol., 102 : 915-922 (1994)]. Ce test permet d'évaluer le potentiel anti-irritant de molécules diverses, sur une lignée cellulaire kératinocytaire humaine (NHEK). Dans ce test on mime une situation irritante en exacerbant la production d'IL-8 des NHEK par l'ajout de rétinol dans le milieu de culture, Il-8 étant impliquée dans l'initiation de l'irritation kératinocytaire. On mesure, ensuite, l'effet anti-irritant d'un composé C-glycoside par son action inhibitrice vis à vis de cette production exacerbée.

### - Conditions expérimentales:

Des kératinocytes épidermiques humains normaux sont incubés en présence de rétinol à 10⁻⁶M pendant 24 heures à 37°C. En réponse au rétinol, on mesure la production de l'agent chimiotactique Interleukine 8 (IL-8) par dosage enzymatique au moyen d'un kit de dosage (Elisa D8050) commercialisé par la société R&D. Les valeurs d'absorbance sont mesurées avec un lecteur de microplaques (MRX/Dynatech) selon les procédures fournies avec le kit de dosage. Pour évaluer l'effet protecteur anti-irritant dudit composé C-glycoside, on mesure la production du marqueur chimiotactique IL-8 par les kératinocytes humains en présence de différentes concentrations supérieures ou égales à 0,04% en matière active de composé C-glycoside.

Un essai de contrôle est également réalisé avec la dexamethazone qui est un agent apaisant de référence.

Les résultats sont exprimés en pourcentage des valeurs contrôles après soustraction du bruit de fond et en pourcentage d'inhibition de ces valeurs obtenues en présence des composés.

**Dosage d'IL8 sans stimulation**

| Traitement | Concentration | IL8 (pg/ml) | % témoin | p | % viabilité |
|---|---|---|---|---|---|
| Témoin | - | 272,1 | 100 | - | 100 |
| C-β-D-xylopyranoside-2-hydroxy-propane | 2% | 145,2 | 53 | p<001 | 97 |

**Dosage d'IL8 + stimulation par le rétinol**

| Traitement | Concentration. | IL8 (pg/ml) | % témoin (+rétinol) | P | % viabilité |
|---|---|---|---|---|---|
| Témoin (+ rétinol) | - | 206,6 | 100 | - | 98 |
| Contrôle sans rétinol | | 129,6 | 63 | P < 0,01 | 100 |
| Dexaméthazone (+ rétinol) | 10⁻⁷M | 117,7 | 57 | P < 0,01 | 99 |
| C-β-D-xylopyranoside-2-hydroxy-propane (+ rétinol) | 2% | 124,4 | 60 | P < 0,01 | 104 |

Ces résultats montrent bien que le C-β-D-xylopyranoside-2-hydroxy-propane diminue la réaction irritante du rétinol.

### Exemple 2 : Sérum

| Nom chimique | % en poids |
|---|---|
| Acide Lactique | 10 |
| Glycérine | 3 |
| C-β-D-xylopyranoside-2-hydroxy-propane en solution à 30 % en poids dans un mélange eau/propylèneglycol 60/40 | 3 (MA) |
| Alcool éthylique 96 degrés non dénaturé | 10 |
| Eau déminéralisée stérilisée | Qsp 100 |

### Exemple 3 : émulsion H/E

| Phase | Nom chimique | % en poids |
|---|---|---|
| A | conservateur | qs |
| | Sel di-sodique de l'acide éthylène di-amine tétracétique, 2 H2O | 0,15 |
| | glycérine | 3 |
| | C-β-D-xylopyranoside-2-hydroxy-propane en solution à 30 % en poids dans un mélange eau/propylèneglyco 60/40 | 3 (MA) |
| | Eau déminéralisée stérilisée | 55.22 |
| B | Mélange de mono-stéarate de glycéryle, stéarate de polyéthylène glycol (100 OE) (50/50) Arlacel 165 | 0,3 |
| | Alcool cétylique pur, d'origine naturelle | 0,4 |
| | conservateur | qs |
| C | Rétinol | 1 |
| | N-lauroylsarcosinate d'isopropyle ou ELDEW SL-205 d'Ajinomoto | 10 |
| D | Copolymère acide acrylique/méthacrylate de stéaryle polymérisé dans un mélange acétate d'éthyle/cyclohéxane : CARBOPOL 1382, NOVEON | 0,5 |
| | Cyclohéxa diméthylsiloxane (viscosité : 8 CST) | 5 |
| E | Eau déminéralisée stérilisée | 10 |
| | Alcool éthylique 96 degrés non dénaturé | 5 |
| F | Copolymère acrylamide/acrylamido 2-méthyl propane sulfonate de sodium en émulsion inverse à 40% dans isoparaffine/eau : Sepigel 305 de Seppic | 1 |
| H | Triéthanolamine à 99 % | 1,03 |
| | Eau déminéralisée stérilisée | 7 |

### Exemple 4 : Gel Apaisant Après soleil

| Phase | Nom chimique | % en poids |
|---|---|---|
| A | | |
| | glycérine | 7 |
| | Allantoine | 1 |
| | conservateur | qs |
| | sequestrant | qs |
| | C-β-D-xylopyranoside-2-hydroxy-propane en solution à 30 % en poids dans un mélange eau/propylèneglycol 60/40 | 3 (MA) |
| | eau | Qsp 100 |
| B | vaseline | 10 |
| | Mélange de cocoyl polyglucoside et d'alcool cetylique, stearylique Montanov 82 de Seppic | 3 |
| | conservateur | qs |
| | Huile végétale | 3 |
| | Cyclopentasiloxane | 5 |
| | Vitamine E | 0,1 |
| C | Gomme de Xanthane | 0,2 |
| | Acide polyacrylamidomethyl propane sulfonique neutralisé partiellement ou Hostacerin AMPS de Clariant | 0,5 |

### Exemple 5 : Gel exfoliant transparent

| Phase | Nom chimique | % en poids |
|---|---|---|
| A | Copolymère acrylamido-2-méthyl propane sulfonate de sodium / hydroxyethylacrylate ou SEPINOV EMT 10 | 2 |
| | glycérine | 4 |
| | Propylène glycol | 4 |
| | sequestrant | qs |
| | Conservateur | qs |
| | colorant | qs |
| | alcool | 10 |
| | C-β-D-xylopyranoside-2-hydroxy-propane en solution à 30 % en poids dans un mélange eau/propylèneglycol 60/40 | 1,5 (MA) |
| | eau | Qsp 100 |
| B | Acide glycolique | 2 |
| | Eau | 10 |
| C | Parfum | qsp |

### Exemple 6 : Déodorant crème

| Phase | Nom chimique | % en poids |
|---|---|---|
| A | Alcool cetylstearylique (C16/C18 30/70) | 3 |
| | Alcool cetylstearylique oxyethylene (33 OE) | 0,5 |
| | Parfum | qs |
| | cyclohexasiloxane | 1 |
| B | conservateur | qs |
| | sequestrant | qs |
| | Hydrochlorure d'aluminium | 15 |
| | C-β-D-xylopyranoside-2-hydroxy-propane en solution à 30 % en poids dans un mélange eau/propylèneglyco 60/40 | 2,4 (MA) |
| | eau | Qsp 100 |

### Exemple 7 : Nettoyant visage

| Nom chimique | % en poids |
|---|---|
| Laurylether sulfate de Na solution aqueuse à 70% | 8 |
| Cocoylbétaine solution aqueuse à 30% | 8 |
| Acide lauryl ether carboxylique (10 OE) | 1 |
| glycérine | 4 |
| Hexylène glycol | |
| Huile végétale | 0,1 |
| Vit E | 0,3 |
| sequestrant | qs |
| conservateur | Qs |
| C-β-D-xylopyranoside-2-hydroxy-propane en solution à 30 % en poids dans un mélange eau/propylèneglycol 60/40 | 1,5 (MA) |
| Parfum | Qs |
| eau | Qsp 100 |

| | |
|---|---|
| MA : matière active. | |

## Revendications

1. Utilisation non thérapeutique d'un dérivé C-glycoside à titre d'agent apaisant.

2. Utilisation selon la revendication 1, destinée à prévenir et/ou traiter les réactions cutanées choisies parmi les rougeurs, les démangeaisons, les échauffements, les sensations de brûlure, de picotement et de tiraillement.

3. Utilisation selon la revendication 1 ou 2, **caractérisée en ce que** ledit dérivé C-glycoside est destiné à prévenir et/ou diminuer ladite réaction cutanée induite par au moins une condition choisie parmi l'action de xénobiotiques, de produits chimiques, de composés susceptibles de provoquer une irritation de la peau, d'un peeling, l'action de la température, du climat, de rayonnements UV, de la pollution atmosphérique, ou encore par les frottements.

4. Utilisation selon la revendication 3, **caractérisée en ce que** ledit dérivé C-glycoside est destiné à prévenir et/ou diminuer l'effet irritant d'une composition cosmétique ou dermatologique contenant un ou plusieurs composés susceptibles de provoquer une irritation de la peau.

5. Utilisation selon la revendication 4, **caractérisée en ce que** ledit dérivé C-glycoside est incorporé dans une composition séparée destinée à être appliquée après une composition contenant le(s) composé(s) susceptible(s) de provoquer une irritation de la peau.

6. Utilisation selon la revendication 4, **caractérisée en ce que** ledit dérivé C-glycoside est incorporé dans une composition contenant le(s) composé(s) susceptible(s) de provoquer une irritation de la peau.

7. Utilisation selon l'une quelconque des revendications 4 à 6, **caractérisée en ce que** le composé susceptible de provoquer une irritation de la peau est choisi parmi les actifs cosmétiques, les actifs dermatologiques, les tensioactifs, les parfums, les solvants, les propulseurs, les conservateurs, les détergents et leurs mélanges.

8. Utilisation selon la revendication 7, **caractérisée en ce que** le composé susceptible de provoquer une irritation de la peau est un actif cosmétique ou dermatologique choisi parmi le groupe des agents desquamants, des rétinoïdes, de la vitamine D et de ses dérivés, de la vitamine B9 et de ses dérivés, de l'urée et ses dérivés, des peroxydes, des antichutes, des teintures et des colorants capillaires, des agents anti-transpirants, des actifs dépilatoires et/ou de permanentes, du thioglycolate et de ses sels, du phénoxyéthanol, du 1,2-pentanediol, des solutions alcooliques parfumantes, des anthralines, des anthranoïdes, des sels de lithium, des dépigmentants, des actifs amincissants à effet chauffant, des nicotinates et de leurs dérivés, de la capsaïcine, des actifs antipoux, des antiprolifératifs, des agents antiviraux, des antiparasitaires, des antifongiques, les antiprurigineux, des antiséborrhéiques, des filtres solaires, des propigmentants et de leurs mélanges ou bien est un agent tensioactif anionique, cationique ou amphotère.

9. Utilisation selon la revendication 8, **caractérisée en ce que** le composé susceptible de provoquer une irritation de la peau est choisi parmi les rétinoïdes, les α-hydroxyacides, les β-hydroxyacides, les acides dicarboxyliques saturés et insaturés tels que l'acide octadécène dioique, les tensioactifs anioniques, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique, l'acide cinnamique et leurs mélanges.

10. Utilisation selon l'une quelconque des revendications 4 à 9, **caractérisée en ce que** le(s) composé(s) susceptible(s) de provoquer une irritation de la peau est présent dans une teneur allant de 0,0001 à 70 % en poids et de préférence de 0,01 à 50 % en poids par rapport au poids total de la composition.

11. Utilisation non thérapeutique, en particulier cosmétique, d'un dérivé C-glycoside pour le traitement des peaux et/ou cuirs chevelus sensibles.

12. Utilisation selon la revendication précédente, **caractérisée en ce qu'**elle est destinée au traitement des rougeurs et/ou des dartres et/ou des sensations dysesthésiques choisies parmi picotements, fourmillements, démangeaisons, brûlures, échauffements, inconforts, tiraillements des peaux et cuirs chevelus sensibles.

13. Utilisation selon l'une des revendications 11 ou 12, **caractérisée en ce que** le dérivé C-glycoside est associé à un extrait de rose, notamment un extrait de rose tel que détaillé ci-après.

14. Utilisation d'au moins un dérivé C-glycoside pour la préparation d'une composition dermatologique destinée à prévenir et/ou traiter les désordres cutanés liés à une irritation cutanée.

15. Utilisation selon la revendication 14, **caractérisée en ce que** ledit dérivé C-glycoside est destiné à prévenir et/ou diminuer l'effet irritant d'une composition cosmétique ou dermatologique contenant un ou plusieurs composés susceptibles de provoquer une irritation de la peau.

16. Utilisation selon la revendication 14, **caractérisée en ce que** ladite composition est destinée à traiter des désordres cutanés liés à une irritation cutanée de type inflammatoire ou immuno-allergique.

17. Utilisation selon l'une quelconque des revendications 14 à 16, **caractérisée en ce que** les désordres cutanés sont choisis parmi les dartres, les oedèmes et/ou boutons, l'érythème inflammatoire; les prurits; le psoriasis, l'atopie cutanée, la dermatite atopique ; l'urticaire; les dermites de contact, l'eczéma ; les dermatites séborrhéiques ; l'acné ; les hyperpigmentations inflammatoires ; les dermatoses immunes ; les maladies immunes bulleuses ; la sclérodermie ; l'élastose actinique ; les pelades ou *alopecia areata ;* le vitiligo ; le lupus érythémateux systémique ; le *pemphigus vulgaris ;* les épidermolyses bulbeuses dystrophiques et la canitie d'origine autoimmune.

18. Utilisation selon l'une quelconque des revendications précédentes, **caractérisée en ce que** le dérivé C-glycoside répond à la formule générale (I) suivante : 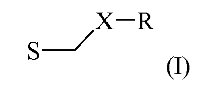 dans laquelle :
- R représente :
- un radical alkyle linéaire, saturé en C₁ à C₂₀, en particulier en C₁ à C₁₀, insaturé en C₂ à C₂₀, en particulier en C₂ à C₁₀, ou un radical alkyle ramifié ou cyclique, saturé ou insaturé, en C₃ à C₂₀, en particulier en C₃ à C₁₀ ;
- un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀;
la chaîne hydrocarbonée constituant lesdits radicaux pouvant, le cas échéant, être interrompue par 1, 2, 3 ou plus d'hétéroatomes choisis parmi :
- un oxygène,
- un soufre,
- un azote, et
- un silicium,
et pouvant être éventuellement substituée par au moins un radical choisi parmi :
- -OR₄,
- -SR₄,
- -NR₄R₅,
- -COOR₄,
- -CONHR₄,
- -CN,
- un atome d'halogène,
- un radical hydrofluoro- ou perfluoro-alkyle, en C₁ à C₆, et/ou
- un radical cycloalkyle en C₃ à C₈,
avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀ , notamment en C₃ à C₁₂ ; ou un radical aryle en C₆ à C₁₀,
- X représente un radical choisi parmi les groupements : 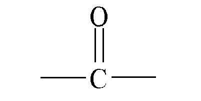 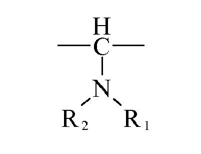 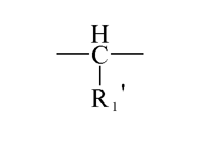 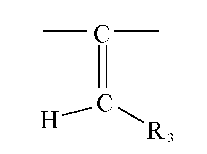 avec R₁, R₂ et R₃ représentant, indépendamment l'un de l'autre, un atome d'hydrogène, ou un radical R, avec R tel que défini précédemment, et R'₁ représentant un atome d'hydrogène, un groupe -OH ou un radical R tel que défini précédemment, R₁ pouvant désigner également un radical aryle en C₆ à C₁₀,
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β,
ainsi que leurs sels cosmétiquement acceptables, leurs solvates tels que les hydrates et leurs isomères.

19. Utilisation selon la revendication 18, **caractérisée en ce que** S représente un monosaccharide choisi parmi le D-glucose, le D-xylose, le L-fucose, le D-galactose, le D-maltose et notamment le D-xylose.

20. Utilisation selon la revendication 18 ou 19, **caractérisée en ce que** X représente un groupement choisi parmi -CO-, -CH(OH)-, -CH(NH₂)-, et préférentiellement un groupement -CH(OH)-.

21. Utilisation selon la revendication 18, 19 ou 20, **caractérisée en ce que** R désigne un radical linéaire en C₁-C₄, notamment C₁-C₃, éventuellement substitué par -OH, -COOH ou -COOR"₂, R"₂ étant un radical alkyle saturé en C₁-C₄, notamment éthyle.

22. Utilisation selon l'une quelconque des revendications précédentes, **caractérisée en ce que** le dérivé C-glycoside est choisi parmi :
- le C-β-D-xylopyranoside-n-propane-2-one,
- le C-α-D-xylopyranoside-n-propane-2-one,
- le C-β-D-xylopyranoside-2-hydroxy-propane,
- le C-α-D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1-(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside) -2-hydroxy-propane,
- le 1-(C-β-D-glucopyranosyl)-2-hydroxyl-propane,
- le 1-(C-α-D-glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

23. Utilisation selon l'une quelconque des revendications précédentes, **caractérisée en ce que** le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

24. Utilisation selon l'une quelconque des revendications précédentes, **caractérisée en ce que** le dérivé C-glycoside est présent en une teneur allant d'environ 0,0001 % à environ 25 % en poids en matière active par rapport au poids total de la composition, et en particulier d'environ 0,001 % à environ 10 % en poids en matière active, et plus particulièrement d'environ 0,05 % à environ 5 % en poids en matière active par rapport au poids total de la composition.

25. Utilisation selon l'une quelconque des revendications 1 à 24, **caractérisée en ce que** le dérivé C-glycoside est associé à au moins un agent anti-inflammatoire, un agent apaisant ou un de leurs mélanges.

26. Utilisation selon l'une des revendications 1 à 25, **caractérisée en ce que** la composition est destinée au traitement des peaux et/ou muqueuses et/ou cuirs chevelus irritables et/ou allergiques.

27. Composition pour application topique sur la peau et/ou les muqueuses et/ou le cuir chevelu comprenant, dans un milieu physiologiquement acceptable, au moins un dérivé C-glycoside et au moins un composé cosmétique ou pharmaceutique susceptible de provoquer une irritation de la peau choisi parmi les agents desquamants, les rétinoïdes, l'urée et ces dérivés, la vitamine D et ses dérivés, la vitamine B9 et ses dérivés, les peroxydes, les antichutes, les teintures et les colorants capillaires, les agents anti-transpirants, les déodorants, les actifs dépilatoires et/ou de permanentes, le thioglycolate et ses sels, le phénoxyéthanol, le 1,2-pentanediol, les anthralines, les anthranoïdes, les sels de lithium, les dépigmentants, certains actifs amincissants à effet chauffant, les nicotinates et leurs dérivés, la capsaïcine, les actifs antipoux, les antiprolifératifs, les agents antiviraux, les antiparasitaires, les antifongiques, les antiprurigineux, les antiséborrhéiques, certaines filtres solaires, les propigmentants, les alkyl sulfates et alkyl éther sulfates et les tensioactifs cationiques ou amphotères.

28. Composition selon la revendication 27, **caractérisée en ce que** le composé susceptible de provoquer une irritation de la peau est choisi parmi les rétinoïdes, les α-hydroxyacides, les β-hydroxyacides, les acides dicarboxyliques saturés et insaturés tels que l'acide octadécène dioique, les tensioactifs, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique, l'acide cinnamique et leurs mélanges.

29. Composition selon l'une quelconque des revendications 27 ou 28, **caractérisée en ce que** le dérivé C-glycoside est présent en une teneur allant d'environ 0,0001 % à environ 25 % en poids en matière active par rapport au poids total de la composition, et en particulier d'environ 0,001 % à environ 10 % en poids en matière active, et plus particulièrement d'environ 0,05 % à environ 5 % en poids en matière active par rapport au poids total de la composition.

30. Composition selon l'une quelconque des revendications 27 à 29, **caractérisée en ce que** la quantité de composé(s) susceptible(s) de provoquer une irritation de la peau est présent dans une teneur allant de 0,0001 à 70 % en poids et de préférence de 0,01 à 50 % en poids par rapport au poids total de la composition.

31. Composition selon l'une quelconque des revendications 27 à 30, **caractérisée en ce qu'**elle comprend en outre au moins un agent anti-inflammatoire, un agent apaisant ou leur mélange.

32. Composition pour application topique sur la peau et/ou les muqueuses et/ou le cuir chevelu comprenant, dans un milieu physiologiquement acceptable :
- 0,00001 à 95 % en poids d'au moins un composé anti-inflammatoire et/ou apaisant et,
- au moins un dérivé C-glycoside.

33. Composition selon la revendication 31 ou 32, **caractérisée en ce que** le composé anti-inflammatoire est choisi parmi les extraits d'algues capable de moduler la production des cytokines par les kératinocytes, les extraits d'Aloe vera, l'extrait d'écorces et les racines de terminalia sericea et le composé apaisant est choisi parmi l'allantoine, l'acide bêta-glycyrrhétinique, les extraits en contenant comme par exemple l'extrait de Glycyrrhiza Glabra et les complexes en contenant comme le complexe allantoïne/acide glycyrrhétinique ; les planctons, lyophilisés ou non, leurs extraits et leurs complexes; les eaux et extraits de fleurs et de plantes : eaux de camomille, de tilleul, rose, extraits de bouleau ; le bisabolol ; les huiles essentielles par exemple de coriandre ; les algues notamment du type Laminaria tel que l'extrait d'algue brune Padina Pavonica ; l'acide acexamique et l'acide transexamique ; l'acide ursolique et les extraits en contenant comme l'extrait de feuille de romarin; les polysaccharides contenant du fucose ; les électrolytes; les acides aminés et les sels divalents de magnésium tel que le magnésium gluconate.

34. Composition selon la revendication 33, **caractérisée en ce que** le composé apaisant est un extrait de rose.

35. Procédé cosmétique pour le soin non thérapeutique de la peau, des cheveux et/ou du cuir chevelu, **caractérisé en ce qu'**il comprend au moins une étape consistant à appliquer sur la peau, les cheveux et/ou le cuir chevelu une composition cosmétique telle que définie selon l'une quelconque des revendications 27 à 34.

36. Procédé cosmétique selon la revendication 35, **caractérisé en ce que** la composition est appliquée sur une peau, une muqueuse et/ou un cuir chevelu irritable et/ou allergique.

37. Procédé cosmétique selon l'une des revendications 35 ou 36, **caractérisé en ce qu'**il est destiné à prévenir et/ou traiter les signes du vieillissement cutané, et/ou à éclaircir la peau, et/ou à raviver l'éclat du teint, et/ou à stimuler la croissance des cheveux ou freiner leur chute.
